# NEW EUROPEAN PATENT SPECIFICATION

(11) **EP 0 516 441 B2**
(45) Date of publication and mention of the opposition decision: **01.04.2009**
(45) Mention of the grant of the patent: 15.04.1998
(21) Application number: 92304881.3
(22) Date of filing: 29.05.1992
(51) Int. Cl.: C07C 1/04

(54) **Process for the conversion of natural gas into higher hydrocarbons**
Verfahren zur Umsetzung von Erdgas in höhere Kohlenwasserstoffe
Procédé pour la conversion de gaz naturel en hydrocarbures supérieurs

(30) Priority: 30.05.1991 US 707477
(43) Date of publication of application: 02.12.1992
(73) Proprietor: BP p.l.c., London EC2M 7BA (GB)
(72) Inventor: Hardman, Stephen, The British Petroleum Co. p.l.c., Sunbury-on-Thames, Middlesex TW16 7LN (GB); Woodfin, William T., The British Petroleum Co. plc, Sunbury-on-Thames, Middlesex TW16 7LN (GB); Ruhl, Robert C., Cleveland Heights, Ohio 44106 (GB)
(74) Representative: Collins, Frances Mary

(56) References cited:
- EP-A2- 0 142 887
- EP-A2- 0 142 888
- US-A- 4 640 766
- US-A- 4 833 170
- VAN RENSBURG S.T.J. DR., PROCESS MANAGER, MOSSGAS PROJECT: 'The Mossgas, Gas to Fuels Process' CHEMSA February 1990, pages 41 - 42
- CHANENCHUK C.A. ET AL: 'The Fischer-Tropsch Synthesis with a Mechanical Mixture of a Cobalt Catalyst and a Copper-Based Water Gas Shift Catalyst' AMERICAN CHEMICAL SOCIETY vol. 5, 1991, pages 847 - 855
- C.N. Satterfield, Slurry Phase Fischer-Tropsch Synthesis: Cobalt Plus a Water-Gas-Shift Catalyst, 1989
- YATES I.C. ET AL: 'Slurry Phase Fischer-Tropsch Synthesis Cobalt Plus Water-Gas 0hift Catalyst, Report to the U.S. Department of Energy for July 1, 1989 to September 30, 1989' 1989,
- YATES I.; SATTERFIELD C.N.: 'Slurry Phase Fischer-Tropsch Synthesis: Cobalt Plus a Water-Gas-Shift Catalyst, Report to the U.S. Department of Energy' 1988,
- C. N. Satterfield, Slurry Phase Fischer-Tropsch Synthesis: Cobalt Plus a Water-Gas-Shift Catalyst, 24.09.1990

## Description

This invention relates to an improved natural gas conversion process.

Much literature is available on the conversion of natural gas into higher hydrocarbons. One possible scheme converts natural gas into synthesis gas, a mixture of hydrogen and carbon monoxide, by reaction with steam and optionally oxygen. This process is known as reforming. The synthesis gas can then be converted into higher hydrocarbons using the Fischer-Tropsch process.

Use of natural gas, typically a mixture of predominantly methane with some higher hydrocarbons, nitrogen and carbon dioxide, as the only carbon-containing material in the feed to the reformer, leads to a synthesis gas with a CO:H₂ molar ratio which is not optimum for use in the Fischer-Tropsch reaction. Accordingly, it is normal practice to remove carbon dioxide co-produced during the reforming process and recycle the desired quantity back to the reformer. The addition of this carbon dioxide to the feed changes the CO:H₂ molar ratio. Careful control of the amount of recycled carbon dioxide allows a desirable CO:H₂ ratio to be achieved. This process is descried, for example, in a review article by Goff and Wang in Chemical Engineering Progress, August 1987, pp. 46-53.

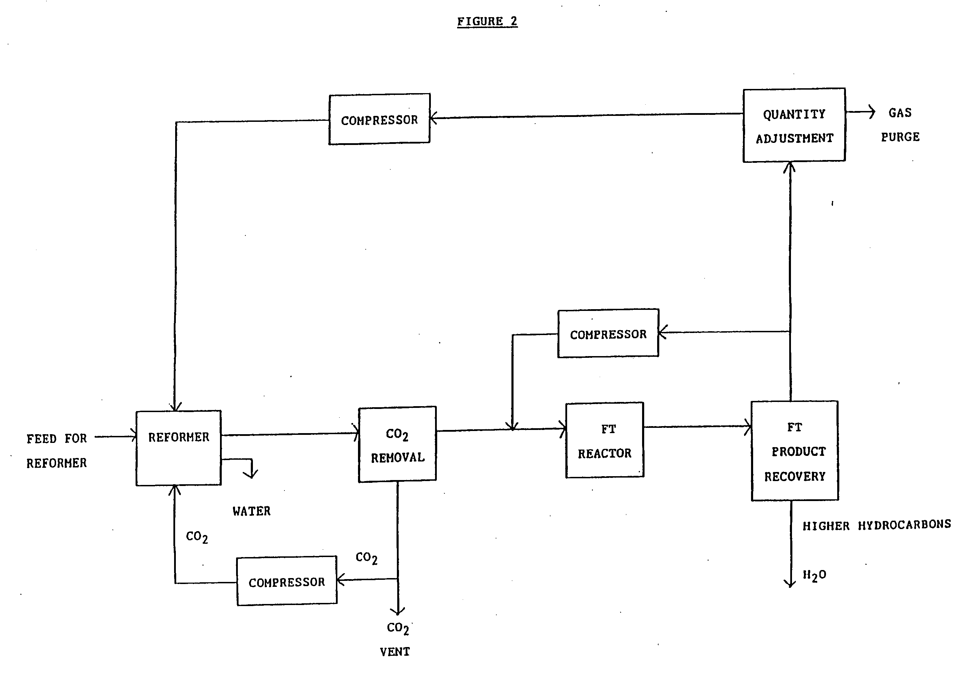

Integrating a conventional reforming process as described above with a conventional Fischer-Tropsch process would result in a reaction scheme such as the one shown in Figure 2 and described later in this specification. GB-A-2183672 describes a process for producing liquid hydrocarbons from a hydrocarbonaceous feed which comprises the following steps:
(i) catalytically reforming at least part of the hydrocarbonaceous feed at elevated temperature and pressure with steam in at least one reforming zone;
(ii) heating the reforming zone(s) by means of a carbon dioxide-containing heating gas comprising a product obtained by partial oxidation of reformer product obtained in step (i) or of a remaining part of the hydrocarbonaceous feed or of a mixture thereof with an oxygen-containing gas in an oxidation zone;
(iii) separating carbon dioxide from heating gas obtained in step (ii);
(iv) catalytically converting at least part of the reformer product obtained in step (i) and/or gas obtained after separating off carbon dioxide in step (iii) at elevated temperature and pressure into normally liquid hydrocarbons; and
(v) combining at least part of the carbon dioxide obtained in step (iii) with hydrocarbonaceous feed for at least one of steps (i) and (ii).

This process adopts the conventional approach described above involving the separation of carbon dioxide before carrying out the conversion of the reformer product (synthesis gas) into liquid hydrocarbons.

EP-A-142 887 describes a 2-stage process in which steam reforming is performed in the presence of recycled carbon dioxide, followed by a Fischer Tropsch reaction performed in the presence of carbon dioxide and a special catalyst with cobalt and at least one of zirconium, titanium, ruthenium and/or chromium on a silica, alumina or silica/alumina carrier. After the reaction, C₅+ hydrocarbons are separated from gases comprising carbon monoxide, carbon dioxide and hydrogen, at least a portion of which is recycled to the steam reforming.

We have now found an improved scheme for integrating a reforming process with a Fischer-Tropsch process.

Accordingly, the present invention provides a process for the conversion of natural gas into higher hydrocarbons, which comprises the following steps:
(i) reacting natural gas with steam in at least one reforming zone containing a reforming catalyst to produce a first product stream including carbon monoxide, carbon dioxide and hydrogen;
(ii) passing said first product stream, without separating said carbon dioxide, to a Fischer-Tropsch reactor containing a Fischer Tropsch catalyst which is cobalt or iron on a titania, ceria, zirconia or zinc oxide support, to produce a second product stream including hydrocarbons and carbon dioxide;
(iii) passing said second product stream to a recovery zone where the desired higher hydrocarbon products are recovered, the remaining components of said second product stream forming a third product stream; comprising carbon dioxide and methane
(iv) recycling from 50 to 99% vol of the third product stream into the reforming zone of process step (i) such as to give a quantity of carbon dioxide of from 10 to 40%v based on the natural gas feed.

The reforming step (i) is well known in the art. The reaction of natural gas with steam is known as steam reforming, while the reaction of natural gas with steam in the additional presence of oxygen is known as autothermal reforming. Either steam reforming or autothermal reforming, or a combination of both, may be used in step (i).

Specific combinations of steam reforming and autothermal reforming are known. In series reforming, the product from a steam reformer is passed to an autothermal reformer along with fresh natural gas and oxygen feed. In convective reforming, steam and natural gas are partially reacted, and the product is passed to an autothermal reformer along with fresh natural gas, steam and oxygen feed. The product stream from the autothermal reformer, which is at a very high temperature, is circulated back to the initial reactor where it is passed outside the reaction zone to provide a source of heat for the reaction. Any of these arrangements may be used in the process of the present invention. Throughout this specification and claims, except where the context otherwise requires, the term "reformer" includes one or more than one reformer; where there is more than one reformer, these may be optionally of different types.

The reforming reaction is preferably carried out at a temperature in the range of from 700 to 1100, especially 780 to 1050°C. The pressure is preferably in the range of from 1000 to 8000 kPa (10 to 80 barg), especially 2000 to 4000 kPa (20 to 40 barg). Any suitable reforming catalyst, for example a nickel catalyst, may be used.

The output from the reformer includes carbon dioxide. It is necessary, in order to produce a synthesis gas with the desired CO:H₂ ratio, that the feed to the reformer includes carbon dioxide. In conventional practice, this carbon dioxide is obtained by separation of carbon dioxide from the reformer output, and recycle back to the reformer. It is an important feature of the present invention that this separation of carbon dioxide need not be carried out. Separation of carbon dioxide is expensive. It is often carried out by amine stripping. This involves reaction with an amine, followed by removal by boiling, and compression to reach the necessary pressure for recycle back to the reformer. It is a major cost advantage of the process of the invention that this step is unnecessary.

The output from one reforming zone may if desired be passed on to a further reforming zone, but carbon dioxide is not separated from the product stream emerging from the end of the total reforming process. Rather, the first product stream including carbon dioxide is passed into the Fischer-Tropsch reactor. This is an unconventional feedstock for a Fischer-Tropsch reactor, for reasons which will be explained later. The carbon dioxide required in the feedstock for reforming step (i) is obtained by separation from the second product stream issuing from the Fischer-Tropsch reactor.

Preferably, water is separated from the first product stream by cooling before the stream is fed to the Fischer-Tropsch reaction. If the first product stream has a ratio of hydrogen to carbon dioxide above that desired for input to the Fischer-Tropsch reactor, it may be subjected to a suitable hydrogen removal step; for example, the first product stream may be passed to a membrane separation unit to remove excess hydrogen.

Fischer-Tropsch reactors are often operated with a major recycle of unconverted synthesis gas separated from the product stream and introduced with the fresh feedstock. If this is done, it is necessary to remove carbon dioxide from the fresh synthesis gas feed, to prevent it building up to unacceptably high standing concentrations in the recycle loop. In the present invention, at least a portion of the third product stream is fed to the reformer rather than being recycled to the Fischer-Tropsch reactor. Accordingly, there is no excessive build up of carbon dioxide in the Fischer-Tropsch reactor, and use of the carbon dioxide containing feedstock from the reformer is accordingly acceptable. The proportion of the third product stream fed to the reformer is such as to achieve the desired quantity of carbon dioxide in the feed for the reformer. This quantity will of course depend on a number of factors including the temperature and pressure used as well as the content of higher hydrocarbons. A major factor determining the quantity of the recycle is the content of inert materials, such as nitrogen. Natural gas usually contains nitrogen, and unless some of this nitrogen is removed, it will reach unacceptable levels in the system. Accordingly, some of the third product stream is normally removed in a gas purge which may be utilised as fuel. At least 50%, especially at least 75%, up to 99%, of the third product stream is recycled. The quantity of carbon dioxide fed in this recycle is preferably in the range of from 10 to 40%v based on the natural gas feed.

If the feedstock to the reformer contains significant quantities of higher hydrocarbons, this can result in excessive coking of the catalyst. It is conventional to process the feed for a reformer by a pre-reforming step to remove these hydrocarbons. In the process according to the present invention, the portion of the third product stream recycled to the reformer may also if desired be subjected to such a pre-reforming step.

Fischer-Tropsch conditions are well known to those skilled in the art. Preferably, the temperature is in the range of from 150 to 350°C, especially 180 to 240°C, and the pressure is in the range of from 100 to 10,000 kPa (0 to 100 barg), especially 1000 to 5000 kPa (10 to 50 barg). The Fischer-Tropsch catalyst comprises cobalt or iron on titania, ceria, zirconia or zinc oxide. The support may itself have some catalytic activity. Preferably the catalyst contains from 2 to 25%w, especially from 5 to 15%w of cobalt or iron. Active metal catalytic components or promotors may be present as well as cobalt or iron if desired.

The useful products obtained from the Fischer-Tropsch reactor will of course depend on the catalyst and the operating conditions used. The initial products may undergo further processing to obtain the desired products; for example, if the initial products obtained include waxy hydrocarbons, these may be cracked using known techniques to produce higher value liquid products, for example in the gasoline or middle distillates range.

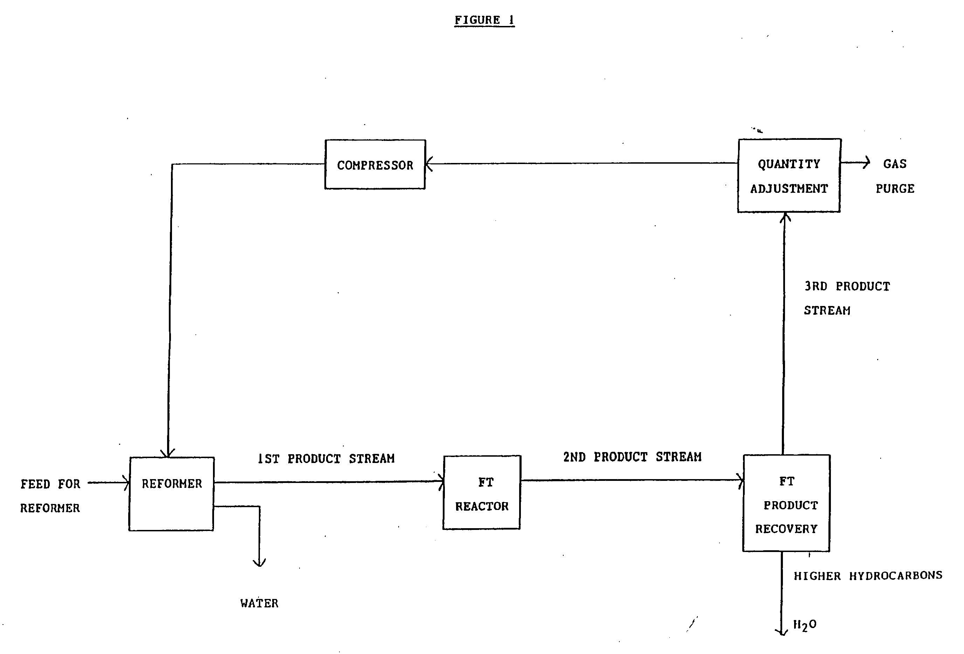

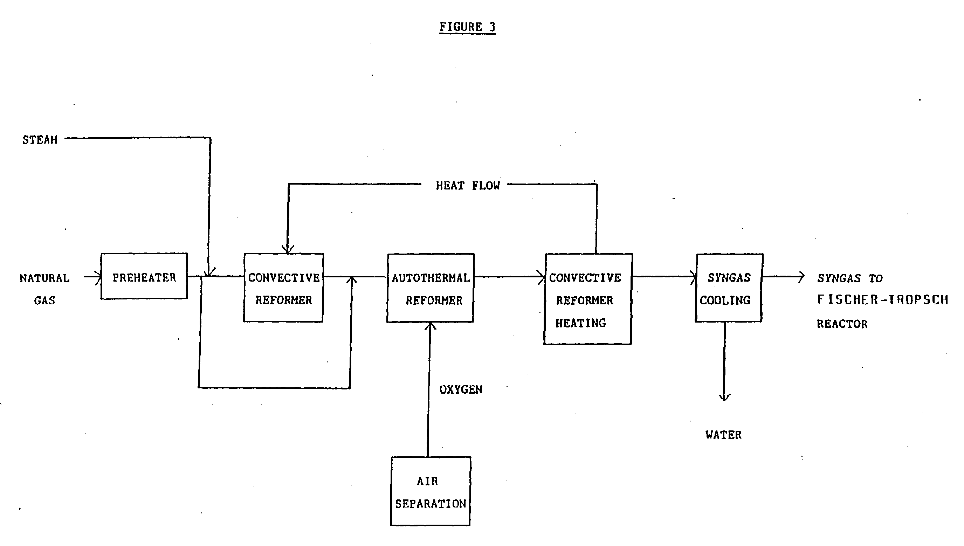

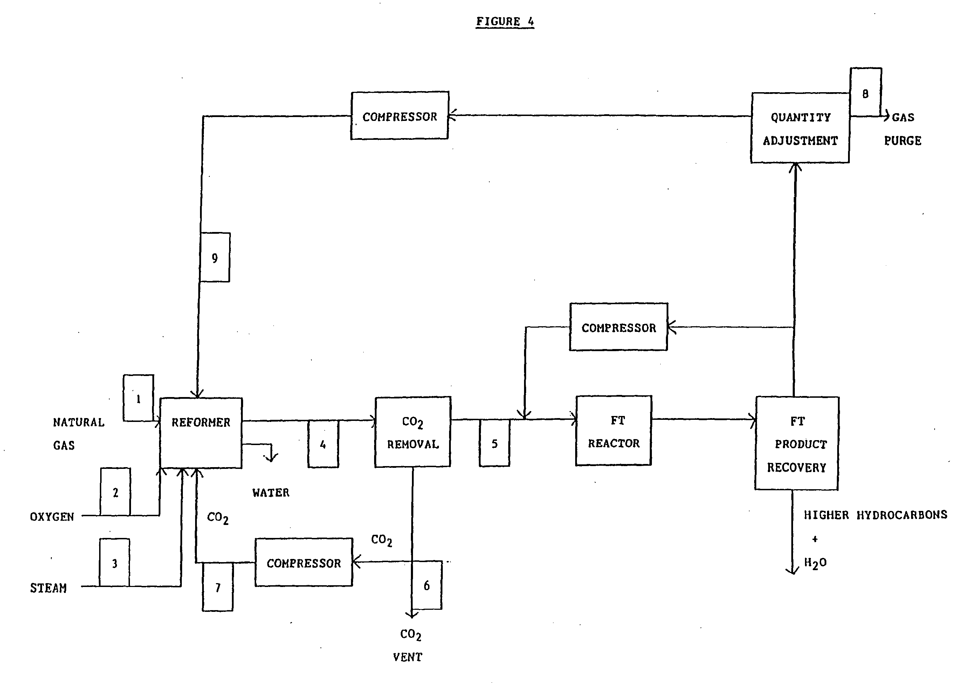

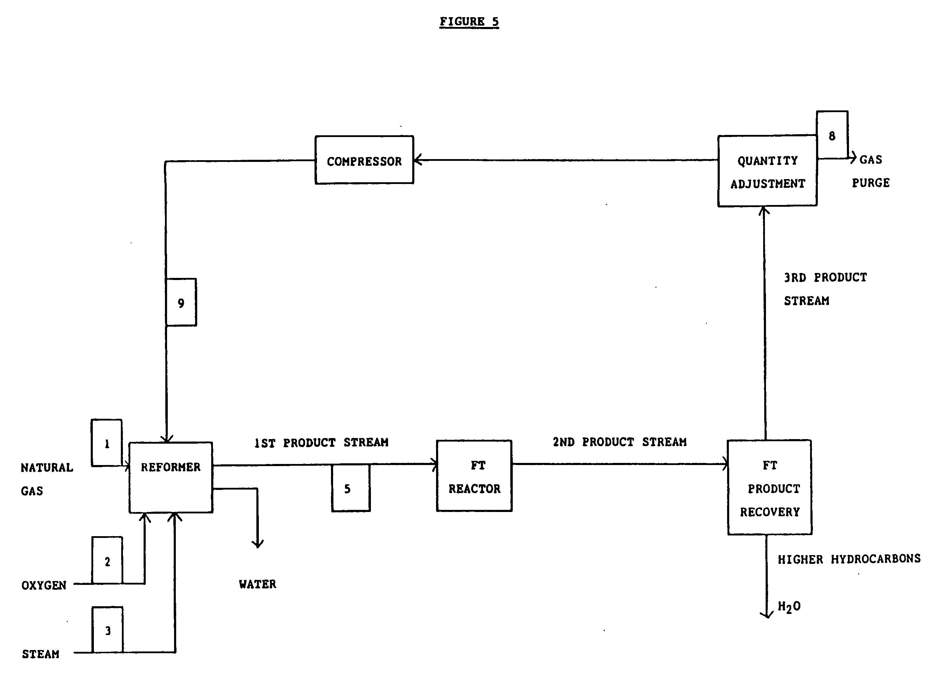

The invention will now be further described with reference to the accompanying drawings, in which Figures 1 and 5 are flow sheets representing a process according to the invention; Figures 2 and 4 are comparison flow sheets representing a typical process operating on prior art principles; and Figure 3 shows a typical reformer arrangement which may be used in the process according to the invention.

In Figure 1, steam and natural gas, optionally together with oxygen, are fed into a reformer. The reformer may be a single reforming zone containing a reforming catalyst, or it may be a system containing more than one reforming zone, the zones being connected in any desired way. The output from the reformer is a first product stream which preferably after condensing and removing most of the water content, is passed directly to a Fischer-Trospsch (FT) reactor operating under Fischer-Tropsch conditions. The output from the Fischer-Tropsch reactor is a second product stream from which the desired liquid or solid hydrocarbon products are removed normally in distillation columns. Low boiling byproducts such as water are also removed at this stage. The third product stream remaining after product recovery contains various gaseous components such as carbon dioxide, carbon monoxide, hydrogen and methane. The third product stream is monitored for carbon dioxide content, and from 50 to 99%vol of the stream is recycled via a compressor to the reformer. The undesired portion of the stream is removed in a gas purge.

By comparison, Figure 2 shows a typical process operating on prior art principles. The process of Figure 2 contains all of the features of Figure 1, but a number of additional process steps are necessary. The output stream from the reformer is subject to a carbon dioxide removal step. The quantity of carbon dioxide required as feed for the reformer is recycled to the reformer via a compressor, while excess carbon dioxide is vented. The remaining product stream is fed to the Fischer-Tropsch reactor. Products from the Fischer-Tropsch reactor are recovered as in Figure 1, but the remaining gas stream is split into two. One part is recycled to the Fischer-Tropsch reactor via a compressor. As much as required of the second part is recycled via a compressor to the reformer. The undesired portion is removed in a gas purge.

Figure 3 shows one possible arrangement for the reformer shown in Figure 1. Natural gas is passed through a preheater, and then split into two streams. One stream is mixed with steam and passed into the reaction zone of a convective reformer. The product emerging from the reaction zone is then mixed with the second stream of natural gas and with oxygen obtained via an air separator, and passed into the reaction zone of an autothermal reformer. The product emerging from the autothermal reformer is a very hot synthesis gas-containing stream, and this is then passed into the heating jacket of the convective reformer referred to above. Here, the hot synthesis gas-containing stream heats the gases entering the reaction zone of the convective reformer. In this process, the synthesis gas-containing stream is cooled; it subsequently undergoes further cooling, water is removed by condensation, and the stream is passed to the Fischer-Tropsch reactor.

Operation of the process according to the invention can be modelled quantitatively, and an example of such modelling is given below.

### (a) TYPICAL CASE WITH CO₂ RECYCLE (conventional process)

The reformer arrangement used for this example is as shown in Figure 3 and the overall process arrangement is as shown in Figure 4.

Part of the natural gas feed (stream 1) is combined with the hydrocarbon recycle stream (stream 9), the CO₂ recycle stream (stream 7), and steam (stream 3) and passed to the primary (convective) reformer where the hydrocarbons are partially reformed. For the purpose of this example the level of steam added is based on providing a (steam + CO₂):carbon ratio in the feed gas of 3.3, which to those skilled in the art represents an acceptable operating regime to prevent carbon laydown on the reforming catalyst. The partially reformed gas is then combined with the remaining natural gas and the reforming is completed by combining with oxygen (stream 2) in a secondary (autothermal) reformer. The synthesis gas produced is partially cooled as it passes back through the convective reformer and some of the sensible heat is used to provide the endothermic heat of reaction for the primary steam reforming. The synthesis gas is cooled further and condensed water is removed. CO₂ is then removed from the synthesis gas before passing to the Fischer-Tropsch conversion stage. Some of the CO₂ removed is compressed and recycled to be co-mixed with the fresh natural gas feed to the convective reformer. The amount of CO₂ recycled depends upon the CO/H₂ ratio required in the downstream process; for a given natural gas composition increasing the amount of CO₂ recycled increases the CO/H₂ ratio (and vice versa).

The cost of removing and recycling the CO₂ is expensive and typically would represent around 30% of the costs associated with producing the synthesis gas within the process.

### (b) TYPICAL CASE WITHOUT CO₂ RECYCLE (according to the invention)

The overall process arrangement for this example is shown in Figure 5. The process assumptions and conditions are the same as in the above example except in the way that CO₂ is recycled to the reformer to adjust the CO/H₂ synthesis gas ratio. In this scheme the synthesis gas is passed directly from the reforming stage (after any necessary cooling/water removal) to the Fischer-Tropsch reaction. The resulting gases are cooled and condensed hydrocarbon and aqueous products are separated from the gas stream. Part of this process tail gas is recycled to the fresh reformer feed. It is a crucial part of the current scheme that sufficient tail gas is recycled to provide the necessary CO₂ feed to the reformer to achieve the desired H₂/CO ratio. Any surplus tail gas is purged from the process and can if required be used as fuel gas for any heating or power generation requirements.

Although the scheme increases the hydrocarbon recycle (stream 9) leading to higher recycle compressor costs and an increased reformer duty, the resultant increases in equipment costs are substantially outweighed by the savings in CO₂ removal and recycle. Overall the nett saving in plant capital (synthesis gas generation and hydrocarbon recycle) is estimated to be about 10-12% between the two schemes outlined above.

The estimated quantities of the materials present at various points in the two schemes (a) and (b) above are given in Table 1. The Table shows that the carbon dioxide can be handled according to the invention without incurring significant penalties in other aspects of process operation. It also shows, surprisingly, that the oxygen requirements for the process do not increase relative to the requirements for the conventional operation.

**Table 1**

| (a) TYPICAL CASE WITH CO₂ RECYCLE (conventional process, Figure 4) | | | | | | | | | |
|---|---|---|---|---|---|---|---|---|---|
| Stream | 1 | 2 | 3 | 4 | 5 | 6 | 7 | 8 | 9 |
| temp (°C) | 400 | 200 | 371 | 50 | 64 | 63 | 44 | 35 | 400 |
| pressure (bar) | 43 | 36 | 40 | 36 | 32 | 1.2 | 143 | 2 | 43 |
| hydrogen | 0 | 0 | 0 | 2329 | 2329 | 0 | 0 | 7 | 0 |
| oxygen | 0 | 583 | 0 | 0 | 0 | 0 | 0 | 0 | 0 |
| nitrogen | 37 | 3 | 0 | 102 | 102 | 0 | 0 | 39 | 59 |
| CO | 0 | 0 | 0 | 1121 | 1121 | 0 | 0 | 8 | 34 |
| CO₂ | 10 | 0 | 0 | 307 | 0 | 89 | 218 | 0 | 0 |
| methane | 895 | 0 | 0 | 30 | 30 | 0 | 0 | 0 | 111 |
| ethane | 57 | 0 | 0 | 0 | 0 | 0 | 0 | 0 | 3 |
| water | 0 | 0 | 1325 | 0 | 0 | 0 | 0 | 0 | 0 |

| (b) SAME CASE WITHOUT CO₂ RECYCLE (according to the invention, Figure 5) | | | | | | | | | |
|---|---|---|---|---|---|---|---|---|---|
| temp (°C) | 400 | 200 | 371 | - | 64 | - | - | 50 | 400 |
| pressure (bar) | 43 | 36 | 40 | - | 32 | - | - | 25 | 43 |
| hydrogen | 0 | 0 | 0 | - | 2470 | - | - | 104 | 270 |
| oxygen | 0 | 586 | 0 | - | 0 | - | - | 0 | 0 |
| nitrogen | 37 | 3 | 0 | - | 143 | - | - | 39 | 104 |
| CO | 0 | 0 | 0 | - | 1189 | - | - | 49 | 130 |
| CO₂ | 10 | 0 | 0 | - | 420 | - | - | 119 | 316 |
| methane | 895 | 0 | 0 | - | 21 | - | - | 26 | 70 |
| ethane | 57 | 0 | 0 | - | 0 | - | - | 0 | 4 |
| water | 0 | 0 | 1850 | - | 0 | - | - | 0 | 0 |
| BASIS 1000 KMOL/HR NATURAL GAS FEED | | | | | | | | | |
| All flows in Kmol/hr | | | | | | | | | |

## Claims

1. A process for the conversion of natural gas into higher hydrocarbons, which comprises the following steps:
(i) reacting natural gas with steam in at least one reforming zone containing a reforming catalyst to produce a first product stream containing carbon monoxide, carbon dioxide and hydrogen;
(ii) passing said first product stream, without separating said carbon dioxide, to a Fischer-Tropsch reactor containing a Fischer Tropsch catalyst, which is cobalt or iron on a titania, ceria, zirconia or zinc oxide support. to produce a second product stream including hydrocarbons and carbon dioxide;
(iii) passing said second product stream to a recovery zone where the desired higher hydrocarbon products are recovered, the remaining components of said second product stream forming a third product stream comprising carbon dioxide and methane;
(iv) recycling from 50 to 99%vol of the third product stream into the reforming zone of process step (i) such as to give a quantity of carbon dioxide of from 10 to 40%v based on the natural gas feed.

2. A process according to claim 1 in which the step (i) is carried out at a temperature in the range of from 700 to 1100°C and a pressure in the range of from 1000 to 8000 kPa.

3. A process according to any one of the preceding claims, in which the Fischer-Tropsch reactor of step (ii) operates at a temperature in the range of from 150 to 350°C and a pressure in the range of from 100 to 10,000 kPa.

4. A process according to any one of the preceding claims, in which step (i) comprises the following steps:
(a) steam and natural gas are partially reacted in a steam reformer and the product is passed to an autothermal reformer along with fresh natural gas, steam and oxygen; and
(b) the product stream from the autothermal reformer is circulated back to the steam reformer where it is passed outside the reaction zone to provide a source of heat for the reaction.

5. A process according to any one of the preceding claims in which water is separated from the first product stream before the stream is fed to the Fischer-Tropsch reactor.

6. A process according to any one of the preceding claims, in which the first product stream is subjected to a hydrogen removal step before the stream is fed to the Fischer-Tropsch reactor.

7. A process according to any one of the preceding claims, in which the portion of the third product stream recycled into the reforming zone of process step (i) is subjected to a pre-reforming step to remove higher hydrocarbons.

## Patentansprüche

1. Verfahren zur Umwandlung von Erdgas in höhere Kohlenwasserstoffe, umfassend nachstehende Schritte:
(i) Umsetzen von Erdgas mit Dampf in zumindest einer Reformingzone, die einen Reformingkatalysator enthält, zur Herstellung eines ersten Produktstroms, der Kohlenmonoxid, Kohlendioxid und Wasserstoff enthält;
(ii) Leiten des ersten Produktstroms ohne Abtrennen des Kohlendioxids zu einem Fischer-Tropsch-Reaktor, der einen Fischer-Tropsch-Katalysator enthält, welcher Kobalt oder Eisen auf einem Titanoxid-, Ceroxid-, Zirkoniumoxid- oder Zinkoxid-Träger ist,
zur Herstellung eines zweiten Produktstroms, der Kohlenwasserstoffe und Kohlendioxid einschließt;
(iii) Leiten des zweiten Produktstroms zu einer Wiedergewinnungszone, worin die gewünschten höheren Kohlenwasserstoffprodukte gewonnen werden, wobei die übrigen Komponenten des zweiten Produktstroms einen dritten Produktstrom bilden, umfassend Kohlendioxid und Methan;
(iv) Zurückführen von 50 bis 99 Vol.-% des dritten Produktstroms in die Reformingzone von Verfahrensschritt (i), sodass sich eine Menge an Kohlendioxid von 10 bis 40 Vol.-% basierend auf der Erdgasbeschickung ergibt.

2. Verfahren nach Anspruch 1, wobei Schritt (i) bei einer Temperatur im Bereich 700 bis 1100°C und bei einem Druck im Bereich 1000 bis 8000 kPa ausgeführt wird.

3. Verfahren nach einem der vorangehenden Ansprüche, wobei der Fischer-Tropsch-Reaktor von Schritt (ii) bei einer Temperatur im Bereich 150 bis 350°C und einem Druck im Bereich 100 bis 10 000 kPa betrieben wird.

4. Verfahren nach einem der vorangehenden Ansprüche, wobei Schritt (i) nachstehende Schritte umfaßt:
(a) Dampf und Erdgas werden teilweise in einem Dampfreformer umgesetzt und das Produkt wird zusammen mit frischem Erdgas, Dampf und Sauerstoff zu einem autothermalen Reformer geleitet; und
(b) der Produktstrom aus dem autothermalen Reformer wird im Kreis zu dem Dampfreformer zurückgeführt, wo er außerhalb der Reaktionszone geleitet wird, um eine Wärmequelle für die Reaktion bereitzustellen.

5. Verfahren nach einem der vorangehenden Ansprüche, wobei aus dem ersten Produktstrom Wasser abgetrennt wird, bevor der Strom in den Fischer-Tropsch-Reaktor gespeist wird.

6. Verfahren nach einem der vorangehenden Ansprüche, wobei der erste Produktstrom einem Schritt zur Entfernung von Wasserstoff unterzogen wird, bevor der Strom in den Fischer-Tropsch-Reaktor gespeist wird.

7. Verfahren nach einem der vorangehenden Ansprüche, wobei der Anteil des dritten Produktstroms, der zu der Reformingzone von Verfahrensschritt (i) zurückgeführt wird, einem Vorreformingschritt unterzogen wird, damit höhere Kohlenwasserstoffe entfernt werden.

## Revendications

1. Procédé de conversion de gaz naturel en hydrocarbures supérieurs, comprenant les étapes suivantes :
(i) la réaction du gaz naturel avec de la vapeur d'eau dans au moins une zone de reformage contenant un catalyseur de reformage pour produire un premier courant de produits contenant du monoxyde de carbone, du dioxyde de carbone et de d'hydrogène ;
(ii) le passage dudit premier courant de produits, sans séparer ledit dioxyde de carbone, dans un réacteur de Fisher-Tropsch contenant un catalyseur de Fisher-Tropsch, lequel est du cobalt ou du fer sur un support en oxyde de titane, en oxyde de cérium, en zircone ou en oxyde de zinc, pour produire un deuxième courant de produits contenant des hydrocarbures et du dioxyde de carbone ;
(iii) le passage dudit deuxième courant de produits dans une zone de récupération où les produits hydrocarbonés supérieurs souhaités sont récupérés, les composants restants dudit deuxième courant de produits formant un troisième courant de produits comprenant du dioxyde de carbone et du méthane ;
(iv) le recyclage de 50 à 99 % en volume du troisième courant de produits dans la zone de reformage de l'étape (i) du procédé, de manière à donner une quantité de dioxyde de carbone de 10 à 40 % en volume sur la base de la charge d'alimentation en gaz naturel.

2. Procédé selon la revendication 1, dans lequel l'étape (i) est réalisée à une température située dans la plage allant de 700 à 1100 °C et à une pression située dans la plage allant de 1000 à 8000 kPa.

3. Procédé selon l'une quelconque des revendications précédentes, dans lequel le réacteur de Fisher-Tropsch de l'étape (ii) fonctionne à une température située dans la plage de 150 à 350 °C et à une pression située dans la plage de 100 à 10 000 kPa.

4. Procédé selon l'une quelconque des revendications précédentes, dans lequel l'étape (i) comprend les étapes suivantes :
(a) la vapeur d'eau et le gaz naturel sont partiellement mis à réagir dans un reformeur à vapeur d'eau et le produit est passé dans un reformeur autothermique avec du gaz naturel frais, de la vapeur d'eau et de l'oxygène ; et
(b) le courant de produits issu du reformeur autothermique est renvoyé vers le reformeur à vapeur d'eau où il est passé à l'extérieur de la zone de réaction pour fournir une source de chaleur pour la réaction.

5. Procédé selon l'une quelconque des revendications précédentes, dans lequel l'eau est séparée du premier courant de produits avant que le courant soit introduit dans le réacteur de Fisher-Tropsch.

6. Procédé selon l'une quelconque des revendications précédentes, dans lequel le premier courant de produits est soumis à une étape d'élimination de l'hydrogène avant que le courant soit introduit dans le réacteur de Fisher-Tropsch.

7. Procédé selon l'une quelconque des revendications précédentes, dans lequel la partie du troisième courant de produits recyclée dans la zone de reformage de l'étape (i) du procédé est soumise à une étape de pré-reformage pour retirer les hydrocarbures supérieurs.
